# EUROPÄISCHE PATENTSCHRIFT

(11) **EP 2 931 197 B1**
(45) Veröffentlichungstag und Bekanntmachung des Hinweises auf die Patenterteilung: **23.05.2018**
(21) Anmeldenummer: 13792344.7
(22) Anmeldetag: 14.11.2013
(51) Int. Cl.: A61F 13/02

(54) **ZUSCHNEIDBARER WUNDVERBAND**
WOUND DRESSING THAT CAN BE CUT TO SIZE
PANSEMENT DÉCOUPABLE

(30) Priorität: 17.12.2012 DE 102012223399
(43) Veröffentlichungstag der Anmeldung: 21.10.2015
(73) Patentinhaber: Paul Hartmann AG, 89522 Heidenheim (DE)
(72) Erfinder: CROIZAT, Pierre, 89542 Herbrechtingen (DE); ECKSTEIN, Axel, 89522 Heidenheim (DE); HOFSTETTER, Jürgen, 89518 Heidenheim (DE)
(74) Vertreter: DREISS Patentanwälte PartG mbB
(86) Internationale Anmeldenummer: PCT/EP2013/073815
(87) Internationale Veröffentlichungsnummer: WO 2014/095174

(56) Entgegenhaltungen:
- EP-A1- 1 440 667
- DE-U1-202004 017 052
- US-A- 5 520 762
- US-A- 5 902 260
- US-A1- 2006 058 721
- US-A1- 2010 318 013
- US-A1- 2011 118 646
- US-B1- 6 264 976
- US-B1- 6 685 682

## Beschreibung

Die Erfindung betrifft einen zuschneidbaren Wundverband, insbesondere Film- oder Folienverband, für die Verwendung bei der Unterdruckwundtherapie zum luft- und unterdruckdichten Verschließen eines Wundraums, mit einer den luft- und unterdruckdichten Verschluss bewirkenden Flachmaterialschicht, insbesondere auf Film- oder Folienbasis, mit einem auf der wundabgewandten Seite der Flachmaterialschicht vorgesehenen, insbesondere rahmenbildenden und insbesondere streifenförmigen Stützelement, welches die Flachmaterialschicht beim Applizieren des Verbands in flächenhafter Erstreckung stabilisiert, und mit einer auf der wundzugewandten Seite der Flachmaterialschicht vorgesehenen Kleberbeschichtung und mit einer die Kleberbeschichtung bis zum Applizieren des Verbands überfangenden ablösbaren Schutzschicht, und mit nicht an der Kleberbeschichtung anhaftenden, insbesondere tabförmigen oder streifenförmigen, manuell ergreifbaren Anfassbereichen, mittels derer ein Ablösen der Schutzschicht von der Kleberbeschichtung erleichtert ist.

Bei der Unterdrucktherapie von Wunden kommuniziert eine Unterdruck erzeugende Einrichtung über eine Saugleitung mit der Wunde oder dem Wundraum, wobei ein luft- und unterdruckdichter Wundverband zum luft- und unterdruckdichten Verschließen der Wunde und des Wundraums vorgesehen ist, so dass ein Unterdruck im Wundraum herstellbar und Flüssigkeiten aus dem Wundraum in einen Sammelbehälter bei der Unterdruck erzeugenden Einrichtung absaugbar sind.

Der Begriff eines Unterdrucks bezeichnet im Zusammenhang mit der vorliegenden Erfindung einen gegenüber dem Umgebungsluftdruck (atmosphärischer Luftdruck) erniedrigten Luftdruck insbesondere innerhalb eines Wundverbands. Das Material eines Wundverbands zum luft- und unterdruckdichten Verschließen eines Wundraums muss daher so ausgebildet sein, dass es der sich einstellenden Druckdifferenz standzuhalten vermag, damit der Unterdruck im Wundraum überhaupt angelegt und aufrechterhalten werden kann. Im Bereich der Unterdrucktherapie in der Wundbehandlung wird der Unterdruck quantitativ als Druckdifferenz zwischen dem Umgebungsluftdruck und dem unterhalb des Abdeckmaterials angelegten Luftdruck angegeben. Typischerweise beträgt diese Druckdifferenz im Bereich der Unterdrucktherapie höchstens 250 mm Hg (mm Quecksilbersäule) (1 mm Hg = 1 Torr. = 133,322 Pa). Dieser Unterdruckbereich bis höchstens 250 mm Hg hat sich als für die Wundheilung geeignet erwiesen. Ein bevorzugter Unterdruckbereich liegt zwischen 10 und 250 mm Hg.

Wenn vorstehend von luft- und unterdruckdichtem Verschließen des Wundraums die Rede ist, so bedeutet dies nicht, dass keinerlei Gasaustausch zwischen dem Wundraum und seiner Umgebung stattfinden darf. Vielmehr ist mit luft- und unterdruckdichtem Verschließen in diesem Zusammenhang gemeint, dass unter Berücksichtigung der eingesetzten unterdruckerzeugenden Einrichtung der für die Unterdrucktherapie von Wunden erforderliche Unterdruck im Wundraum aufgebaut und aufrechterhalten werden kann. Es können deshalb auch Materialien verwendet werden, die eine geringfügige Gaspermeabilität aufweisen, solange der für die Unterdrucktherapie notwendige Unterdruck aufrechterhalten werden kann. - Typischerweise beträgt die Wasserdampfdurchlässigkeit der den luft- und unterdruckdichten Verschluss bewirkenden Flachmaterialschicht 100 bis 2500 g/m²·24h, insbesondere 500 bis 2000 g/m²·24h, insbesondere 800 bis 1600 g/m²· 24h, insbesondere 1050 bis 1450 g/m²24·h, bestimmt gemäß DIN EN 13726-2 bei 37° und 50 % relativer Luftfeuchte.

Aufgrund der Komplexität von Wunden werden häufig zuschneidbare Verbände eingesetzt, die unmittelbar am Patienten zurecht geschnitten und appliziert werden. Häufig werden auch mehrere zugeschnittene Verbandabschnitte mit mehr oder weniger Überlappung übereinander aufgebracht, um eine für die Unterdruckwundtherapie ausreichende Abdichtung zu erreichen, was häufig auch durch Hautunebenheiten, Schorf etc. erschwert wird. Hinzu kommt, dass gerade schwer heilende Wunden häufig sehr ausladend oder unregelmäßig sind und nicht selten eine projizierte Fläche von größer 100 cm² aufweisen. Hierfür werden zuschneidbare Verbände benötigt.

Es sind Verbände bekannt, die streifenförmige Anfassbereiche zum Ergreifen und Ablösen der Releaseschicht oder Schutzschicht von der Kleberbeschichtung aufweisen; diese Anfassbereiche verlaufen insbesondere mittig über das Produkt und sind durch Rückfaltung eines leistenförmigen Abschnitts der Schutzschicht auf sich selbst gebildet. Des Weiteren sind Verbandmittel im weitesten Sinn bekannt mit einem randseitig vorgesehenen tabförmigen oder griffleistenförmigen Anfassbereich. EP 1 440 667 A1 zeigt einen Wundverband, bei dem auf zwei einander gegenüberliegenden Seiten je ein leistenförmiger Anfassbereich vorgesehen ist. Ähnliche Verbände sind aus US 5,520,762 und US 6,264,976 B1 bekannt. US 2011/0118646 befasst sich in einem Ausführungsbeispiel mit dem Ablösen einer wundabgewandten Handhabungsschicht nach dem Aufbringen des Wundverbands auf die Wunde. US 6,685,682 B1 und US 2010/0318013 A1 zeigen einen Film- oder Folienverband, zum Verschließen eines Wundraums, mit einer Flachmaterialschicht auf Film- oder Folienbasis, mit einem auf der wundabgewandten Seite der Flachmaterialschicht vorgesehenen rahmenbildenden Stützelement, welches die Flachmaterialschicht beim Applizieren des Verbands in flächenhafter Erstreckung stabilisiert, und mit einer auf der wundzugewandten Seite der Flachmaterialschicht vorgesehenen Kleberbeschichtung und mit einer die Kleberbeschichtung bis zum Applizieren des Verbands überfangenden ablösbaren Schutzschicht, und mit zwei auf einander gegenüberliegenden Seiten vorgesehenen nicht an der Kleberbeschichtung anhaftenden manuell ergreifbaren Anfassbereichen, mittels derer ein Ablösen der Schutzschicht von der Kleberbeschichtung erleichtert ist und die einstückig mit der Schutzschicht und quer zur Umfangsrichtung des Verbands nach außen vorstehend ausgebildet sind.

Wenn ausgehend von einem flächenhaft ausladenden zuschneidbaren Verband ein Flächenabschnitt abgeschnitten wird, so stellt sich häufig das Problem, dass dieser Flächenabschnitt keinen manuell ergreifbaren Anfassbereich aufweist, so dass das Ablösen der Schutzschicht von der Kleberbeschichtung erschwert ist und hierbei häufig auch die luft- und unterdruckdichte Flachmaterialschicht in Mitleidenschaft gezogen wird.

Der vorliegenden Erfindung liegt die Aufgabe zugrunde, diesem Problem wirksam zu begegnen.

Erfindungsgemäß wird diese Aufgabe durch einen Wundverband mit den Merkmalen des Anspruchs 1 gelöst.

Auf diese Weise wird erfindungsgemäß für fast alle Situationen des Zuschneidens ausgehend von einem flächenhaft ausladenden Verband sichergestellt, dass wenigstens ein Anfassbereich bei dem von dem Verband abgetrennten zu applizierenden Abschnitt vorhanden ist, um die Schutzschicht von der Kleberbeschichtung ablösen zu können, ohne dass die den luft- oder unterdruckdichten Abschluss bewirkende Flachmaterialschicht in Mitleidenschaft gezogen wird. Insgesamt wird hierdurch eine sehr benutzerfreundliche und insbesondere zeitsparende Applizierung des Verbands ermöglicht. Außerdem kann die Menge an infolge nicht ablösbarer Schutzschicht unverwertbaren Verbandmaterials reduziert werden.

Die Mehrzahl von Anfassbereichen umfasst insbesondere wenigstens 5, insbesondere wenigstens 8, insbesondere wenigstens 10 und weiter insbesondere wenigstens 12 Anfassbereiche, die entlang des Umfangs des Wundverbands verteilt angeordnet sind.

Es erweist sich als vorteilhaft, dass die Anfassbereiche quer zur Umfangsrichtung des Verbands vorstehend ausgebildet sind, also über einen den eigentlichen Verband begrenzenden Umfangsrand vorstehen, da sie so leichter manuell ergreifbar sind.

Es erweist sich als vorteilhaft, dass gewissermaßen verteilt über den gesamten Außenumfang des Verbands Anfassbereiche vorgesehen sind. In weiterer Ausbildung der Erfindung wird vorgeschlagen, dass der Abstand der Anfassbereiche voneinander in Umfangsrichtung des Verbands entlang einer Randkante gemessen wenigstens 15 mm, insbesondere wenigstens 20 mm, insbesondere wenigstens 25 mm, und höchstens 60 mm beträgt.

Das gegenüber der dichtenden Flachmaterialschicht biegesteifer oder verwindungssteifer ausgebildete Stützelement soll die eher dünne film- oder folienartige Flachmaterialschicht in ihrer flächenhaften Erstreckung halten und stabilisieren, um den Verband besser in der bestimmungsgemäß intendierten Weise auf der Körperoberfläche des Patienten applizieren zu können. Eine die gesamte flächenmäßige Erstreckung der dichtenden Flachmaterialschicht überfangende Ausbildung des Stützelements bringt wiederum Probleme mit sich, da hierdurch die dreidimensionale Drapierbarkeit des Verbands beeinträchtigt wird. Hierfür erweist es sich als optimal, wenn das Stützelement gewissermaßen rahmenbildend bezüglich des zu applizierenden Verbands oder Verbandsabschnitts vorgesehen ist.

Ein weiteres Problem, das sich bei Verbänden der hier in Rede stehenden Art beim Zuschneiden, also beim Abtrennen von zu applizierenden Abschnitten des Verbands, häufig stellt, ist die infolge des Abtrennens geringere Stabilität der Flachmaterialschicht des abgetrennten Abschnitts, sofern dort das Stützelement dann nur noch in geringerem Umfang, z.B. nur entlang eines oder zweier Ränder des abgetrennten Abschnitts, vorhanden ist.

Es wird erfindungsgemäß vorgeschlagen, dass das Stützelement von einem Außenumfangsbereich des Verbands nach innen erstreckte streifen- oder stegförmig erstreckte Abschnitte aufweist, die aufeinandertreffen, insbesondere einander kreuzen. Auf diese erfindungsgemäße Weise lässt sich eine verbesserte Stabilisierung des Verbands und hiervon abgetrennter Abschnitte realisieren, da die flächenmäßige Überfangung der dichtenden Flachmaterialschicht durch das Stützelement bei der überwiegenden Anzahl von Schnittkonfigurationen größer ist.

Nach einem weiteren Erfindungsgedanken erweist es sich als vorteilhaft, wenn das Stützelement vorzugsweise mehrere quer zu seiner Erstreckung entlang eines Außenumfangsbereichs und/oder quer zur Erstreckung seiner streifen- oder stegförmig erstreckten Abschnitte verlaufende Schwächungslinien, insbesondere Perforationen, aufweist. An diesen Schwächungslinien lässt sich das Stützelement leicht manuell ergreifen und nach dem Applizieren des Verbands auf die Körperoberfläche von der dichtenden Flachmaterialschicht ablösen.

In Weiterbildung dieses Erfindungsgedankens erweist es sich als vorteilhaft, wenn die Schwächungslinien wenigstens 30 mm, insbesondere wenigstens 40 mm, insbesondere wenigstens 50 mm, insbesondere wenigstens 60 mm, und höchstens 150 mm, insbesondere höchstens 130 mm, insbesondere höchstens 100 mm voneinander beabstandet sind. Dieser Abstand ist im Zweifel in Umfangsrichtung des Verbands entlang einer radial äußeren Randkante des eigentlichen Verbands gemessen.

Die den luft- und unterdruckdichten Verschluss bewirkende Flachmaterialschicht ist vorzugsweise transparent ausgebildet. Sie ist vorzugsweise auf Basis eines wasserunlöslichen Polymers oder von einer Metallfolie oder metallisierten Polymerfolie gebildet. Ihre Dicke beträgt vorzugsweise 10 µm bis 500 µm, insbesondere 25 µm bis 100 µm. Das wasserunlösliche Polymer weist dabei eine Löslichkeit von ≤ 10 mg/l, insbesondere von ≤ 1 mg/l, insbesondere von 0,0001 bis 1 mg/l auf, bestimmt gemäß Säulenelutionsmethode nach EU-Richtlinie RL 67-548-EWG, Anhang V, Kapitel A6. Die Flachmaterialschicht ist insbesondere auf Polyurethan-, Polyester-, Polypropylen-, Polyethylen-, Polyamid-, Polyvinylchlorid- oder Polyorganosiloxanbasis (Silikon) oder Mischungen hieraus ausgebildet.

Die Kleberbeschichtung der Flachmaterialschicht könnte insbesondere rasterartig im weitesten Sinne ausgebildet sein. Im Hinblick auf die intendierte Dichtwirkung erweist es sich aber als vorteilhaft, wenn die Kleberbeschichtung vollflächig auf die Flachmaterialschicht aufgebracht ist. Sie ist insbesondere und vorzugsweise auf Acrylatbasis ausgebildet. Vorzugsweise umfasst die Kleberbeschichtung einen druckempfindlichen Kleber (sogenannter pressure sensitive adhesive = PSA). Es wäre aber auch die Verwendung eines sogenannten schaltbaren Klebers denkbar, der beispielsweise durch UV-Bestrahlung aktiviert wird.

Die ablösbare Schutzschicht, welche die Kleberbeschichtung abdeckt, ist vorzugsweise auf Papier- oder Polymerfilmbasis, insbesondere auf Polyethylen-, Polypropylen- oder Polyesterbasis, ausgebildet.

Die ablösbare Schutzschicht weist vorteilhafterweise eine Antihaftbeschichtung auf, um eine im Wesentlichen widerstandslose Ablösbarkeit der Schutzschicht von der Kleberbeschichtung der dichten Flachmaterialschicht zu ermöglichen. Diese Antihaftbeschichtung ist auf einer oder beiden Oberflächen der ablösbaren Schutzschicht vorgesehen. Sie verleiht dieser Oberfläche typischerweise hydrophobe oder lipophobe Eigenschaften und beruht beispielsweise auf Silikon-Fluorcarbon- oder Öl basis.

Das schon mehrfach erwähnte Stützelement ist vorteilhafterweise auf Papier- oder Polymerfilmbasis, insbesondere auf Polyethylen-, Polypropylen-, Polyester- oder Polyamidbasis, ausgebildet. Das Stützelement weist vorteilhafterweise eine Dicke von 10 - 500 µm, insbesondere von 20 - 250 µm, insbesondere von 25 - 100 µm auf. Auch das Stützelement kann in vorteilhafter Weise eine Antihaftbeschichtung auf seiner der Flachmaterialschicht zugewandten Seite aufweisen, damit das Stützelement nach dem Applizieren des Verbands auf die Körperoberfläche des Patienten leicht abgelöst werden kann, ohne allzu große Schäl- oder Scherkräfte auf die Flachmaterialschicht auszuüben.

Nach einer bevorzugten Ausführungsform der Erfindung wird der Verbund aus dichtender Flachmaterialschicht und Trägerelement dadurch hergestellt, dass die Flachmaterialschicht direkt auf eine Seite des Stützelements aufextrudiert wird. Auch das Stützelement ist vorzugsweise in Form einer kontinuierlichen, also flächenhaft durchgehend erstreckten Schicht hergestellt, wobei dann später durch spezielle Schneidtechniken ("kiss cut") Ausnehmungen in der Stützelementschicht gebildet werden, ohne dass die dichtende Flachmaterialschicht hierbei in Mitleidenschaft gezogen wird.

Weitere Merkmale, Einzelheiten und Vorteile der Erfindung ergeben sich aus den beigefügten Patentansprüchen und aus der zeichnerischen Darstellung und nachfolgenden Beschreibung einer bevorzugten Ausführungsform des erfindungsgemäßen Verbands.

In der Zeichnung zeigt:
Figur 1 eine Draufsicht auf einen erfindungsgemäßen zuschneidbaren Verband; und
Figur 2 eine schematische Schichtenfolge als Schnittansicht mit Schnittebene II-II in Figur 1.

Die Figuren zeigen einen insgesamt mit dem Bezugszeichen 2 bezeichneten zuschneidbaren Wundverband für die Verwendung bei der Unterdruckwundtherapie zum luft- und unterdruckdichten Verschließen eines nicht dargestellten Wundraums. Die Unterdruckkommunikation einer unterdruckerzeugenden Einrichtung mit dem Wundraum erfolgt dadurch, dass ein Leitungsmittel typischerweise und vorzugsweise unter Verwendung einer nicht dargestellten Anschlussvorrichtung durch eine noch auszubildende Durchtrittsöffnung in dem Verband hindurchgeführt wird. Der in den Figuren gezeigte zuschneidbare Verband 2 kann sehr ausladend ausgebildet sein, er kann insbesondere an sich beliebige Längs- oder Querabmessungen aufweisen, wobei der Verband 2 dazu bestimmt und geeignet ist, dass hiervon Abschnitte an sich beliebiger Geometrie abgeschnitten werden, um zum unterdruckdichten Abdecken und Verschließen eines Wundraums verwendet zu werden, wobei der Wundverband oder die Wundverbandabschnitte auf unversehrten Hautoberflächen um die Wunde herum klebend befestigt werden.

Der Wundverband 2 umfasst eine den eigentlichen luft- und unterdruckdichten Verschluss bewirkende Flachmaterialschicht 4, ein auf einer wundabgewandten Seite 6 der Flachmaterialschicht 4 vorgesehenes im beispielhaft dargestellten Fall rahmenbildendes Stützelement 8, eine Kleberbeschichtung 10 auf einer wundzugewandten Seite 12 der Flachmaterialschicht 4 und eine die Kleberbeschichtung 10 überfangende ablösbare Schutzschicht 14. Des Weiteren sind nicht an der Kleberbeschichtung 10 anhaftende Anfassbereiche 16 ersichtlich, die manuell ergreifbar sind und das Ablösen der Schutzschicht 14 von der Kleberbeschichtung 10 erleichtern.

Im beispielhaft und bevorzugt dargestellten Fall ist das Stützelement rahmenbildend ausgebildet, d.h. es erstreckt sich in einer Umfangsrichtung 18 entlang eines äußeren Randbereichs oder Außenumfangsbereichs 20 in Umfangsrichtung 18 vorzugsweise durchgehend. Auf diese Weise ist bereits eine flächenhafte Stabilisierung der gegenüber dem Stützelement 8 eher flächenhaft unstabilen, insbesondere "flimsigen" Flachmaterialschicht 4 gewährleistet. Das Stützelement 8 bzw. dessen Material ist dabei biegesteifer als die dichtende Flachmaterialschicht 4 bzw. deren Material ausgebildet und vermag diese daher zu stabilisieren.

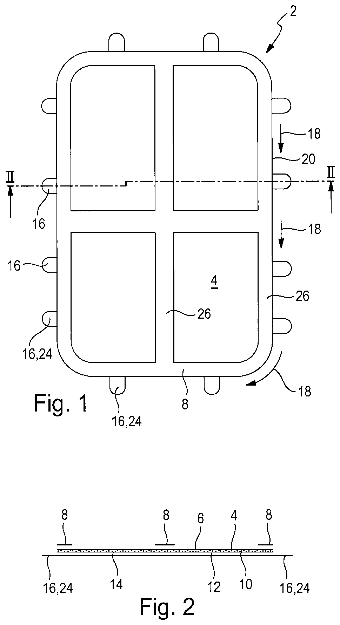

Des Weiteren umfasst das Stützelement 8 aber von dem äußeren Randbereich 20 des Verbands nach innen erstreckte streifen- oder stegförmig erstreckte Abschnitte 22, die im beispielhaft dargestellten Fall mittig aufeinander treffen und sich hierbei kreuzen. Hierdurch wird eine weitere Stabilisierung der Flachmaterialschicht 4 beim Applizieren erreicht. Sofern ausgehend von dem in Figur 1 dargestellten Verband 2 ein mehr oder weniger großer Abschnitt abgeschnitten wird, so dürfte bei diesem Abschnitt das Stützelement 8 nicht nur entlang des äußeren Randbereichs 20 vorgesehen sein, sondern zumindest Teile der nach innen laufenden Abschnitte 22 des Stützelements 8 würden zusätzlich stabilisierend wirken. Ein abgetrennter Abschnitt des Verbands ist daher deutlich besser stabilisiert verglichen mit einer Ausführungsform ohne diese nach innen laufenden streifen- oder stegförmig erstreckten Abschnitte 22. Es sei an dieser Stelle ausdrücklich darauf hingewiesen, dass die in Figur 1 beispielhaft dargestellte Geometrie nicht zwingend ist und zahlreiche Abweichungen oder andere Ausführungen von nach innen laufenden streifen- oder stegförmig erstreckten Abschnitten denkbar und vorteilhaft wären.

Das Stützelement 8 ist lösbar haftend auf der wundabgewandten Seite 6 der dichtenden Flachmaterialschicht 6 vorgesehen. Es kann beispielsweise durch mechanisches Verpressen, durch Wärmelaminierung, mittels einer Klebeschicht oder durch statische Wechselwirkungen lösbar an der Flachmaterialschicht 4 haften. Nach dem Applizieren der Flachmaterialschicht 4 am Patienten wird das Stützelement 8 von der körperabgewandten Seite 6 der Flachmaterialschicht 4 entfernt.

Der Wundverband umfasst des Weiteren eine Vielzahl von Anfassbereichen 16, die über den Außenumfang verteilt, in Umfangsrichtung 18 aufeinander folgend vorgesehen sind. Die Anfassbereiche 16 stehen quer zur Umfangsrichtung 18 des Verbands nach außen vor. Im beispielhaft dargestellten Fall sind diese Anfassbereiche 16 als einstückig nach außen vorstehende Laschen oder Tabs 24 der Schutzschicht 14 gebildet. Es wäre indessen aber auch möglich, dass die Anfassbereiche 16 von hiervon separaten Materialabschnitten gebildet sind, die an die Schutzschicht 14 angefügt sind oder zwischen die Flachmaterialschicht 4 und die Schutzschicht 16 eingefügt sind. Es wäre auch denkbar, dass in Umfangsrichtung 18 über den Außenumfang verteilt eine Mehrzahl von Anfassbereichen vorgesehen ist, die nicht wie dargestellt nach außen vorstehen, sondern innerhalb einer Umfangskontur realisiert sind. In diesem Fall erweist es sich auch als vorteilhaft, wenn die Anordnung dieser Anfassbereiche markiert ist, um einen leichteren gezielteren Eingriff mit einem Finger zu ermöglichen. Da über den gesamten Außenumfang in Umfangsrichtung 18 aufeinander folgend eine Vielzahl von Anfassbereichen 16 vorgesehen ist, weist auch ein abgetrennter Abschnitt des in Figur 1 dargestellten zuschneidbaren Verbands 2 mit hoher Sicherheit einen solchen Anfassbereich 16 auf, wodurch erfindungsgemäß sichergestellt ist, dass die Schutzschicht 14 bei jedem abgetrennten Abschnitt auf einfache Weise ablösbar ist.

Schließlich wird noch auf eine weitere Besonderheit des Verbands 2 hingewiesen. Das Stützelement 8 umfasst mehrere quer zu seiner jeweiligen Erstreckung verlaufende Schwächungslinien 26 insbesondere in Form von Perforationen. Nach dem Applizieren der dichtenden Flachmaterialschicht 4 am Patienten erweist es sich als vorteilhaft, dass im Bereich dieser Schwächungslinien 26 das Stützelement mit den Fingern verhältnismäßig leicht von der wundabgewandten Seite 6 der dichtenden Flachmaterialschicht 4 abgehoben und dann als Ganzes abgelöst werden kann. Es wäre indessen auch denkbar und vorteilhaft, wenn auch das Stützelement 8 manuell ergreifbare und vorzugsweise nach außen vorstehende Anfassbereiche aufweisen würde. Diese Anfassbereiche könnten mit den der Schutzschicht zugeordneten Anfassbereichen in vollständiger oder teilweiser Überdeckung angeordnet sein.

Der erfindungsgemäße zuschneidbare Verband 2 ist im beispielhaft dargestellten Fall rechteckförmig ausgebildet. An seinen jeweiligen gegenüberliegenden geraden Rändern sind beispielhaft jeweils zwei bzw. jeweils vier Anfassbereiche 10 vorgesehen. Die rechteckförmige Gestalt des zuschneidbaren Verbands 2 ist indessen rein beispielhaft und zweckmäßig; andere insbesondere runde oder ovale Ausbildungen sind ebenso denkbar. Für die jeweilige Ausbildung, Dimensionierung und Anordnung der Flachmaterialschicht 4, des Stützelements 8, der Kleberbeschichtung 10, der Schutzschicht 14 und der Anfassbereiche 16 gelten die eingangs gemachten Ausführungen.

## Patentansprüche

1. Zuschneidbarer Wundverband (2), nämlich Film- oder Folienverband, für die Verwendung bei der Unterdruckwundtherapie zum luft- und unterdruckdichten Verschließen eines Wundraums, mit einer den luft- und unterdruckdichten Verschluss bewirkenden Flachmaterialschicht (4) auf Film- oder Folienbasis, mit einem auf der wundabgewandten Seite (6) der Flachmaterialschicht (4) vorgesehenen rahmenbildenden Stützelement (8), welches die Flachmaterialschicht (4) beim Applizieren des Verbands (2) in flächenhafter Erstreckung stabilisiert, und mit einer auf der wundzugewandten Seite (12) der Flachmaterialschicht (4) vorgesehenen Kleberbeschichtung (10) und mit einer die Kleberbeschichtung (10) bis zum Applizieren des Verbands überfangenden ablösbaren Schutzschicht (14), und mit nicht an der Kleberbeschichtung (10) anhaftenden, insbesondere tabförmigen oder streifenförmigen, manuell ergreifbaren Anfassbereichen (16), mittels derer ein Ablösen der Schutzschicht (14) von der Kleberbeschichtung (10) erleichtert ist, wobei in Umfangsrichtung (18) des Verbands über den Außenumfang verteilt eine Mehrzahl von Anfassbereichen (16) vorgesehen ist, die einstückig mit der Schutzschicht (14) ausgebildet sind und quer zur Umfangsrichtung (18) des Verbands nach außen vorstehend ausgebildet sind, und wobei der Abstand der Anfassbereiche (16) voneinander in Umfangsrichtung (18) des Verbands entlang einer Randkante gemessen wenigstens 10 mm und höchstens 80 mm beträgt und wobei das rahmenbildende Stützelement (8) von einem Außenumfangsbereich (20) des Verbands nach innen erstreckte streifen- oder stegförmig erstreckte Abschnitte (22) aufweist und diese streifen- oder stegförmig erstreckten Abschnitte (22) aufeinandertreffen.

2. Verband nach Anspruch 1, **dadurch gekennzeichnet, dass** der Abstand der Anfassbereiche (16) voneinander in Umfangsrichtung des Verbands entlang einer Randkante gemessen wenigstens 15 mm, insbesondere wenigstens 20 mm, insbesondere wenigstens 25 mm, und höchstens 60 mm beträgt.

3. Verband nach Anspruch 1 oder 2, **dadurch gekennzeichnet, dass** die streifen- oder stegförmig erstreckten Abschnitte (22) einander kreuzen.

4. Verband nach einem oder mehreren der vorstehenden Ansprüche, **dadurch gekennzeichnet, dass** das Stützelement (8) vorzugsweise mehrere quer zu seiner Erstreckung entlang eines Außenumfangsbereichs (20) und/oder quer zur Erstreckung seiner streifen- oder stegförmig erstreckten Abschnitte (22) verlaufende Schwächungslinien (26), insbesondere Perforationen, aufweist.

5. Verband nach einem oder mehreren der vorstehenden Ansprüche, **dadurch gekennzeichnet, dass** die Schwächungslinien (26) wenigstens 30 mm, insbesondere wenigstens 40 mm, insbesondere wenigstens 50 mm, insbesondere wenigstens 60 mm, und höchstens 150 mm, insbesondere höchstens 130 mm, insbesondere höchstens 100 mm voneinander beabstandet sind.

6. Verband nach einem oder mehreren der vorstehenden Ansprüche, **dadurch gekennzeichnet, dass** die den luft- und unterdruckdichten Verschluss bewirkende Flachmaterialschicht (4) transparent ausgebildet ist.

7. Verband nach einem oder mehreren der vorstehenden Ansprüche, **dadurch gekennzeichnet, dass** die den luft- und unterdruckdichten Verschluss bewirkende Flachmaterialschicht (4) auf Polyurethan-, Polyester-, Polypropylen-, Polyethylen-, Polyamid-, Polyvinylchlorid- oder Polyorganosiloxanbasis oder Mischungen hieraus ausgebildet ist.

8. Verband nach einem oder mehreren der vorstehenden Ansprüche, **dadurch gekennzeichnet, dass** die Kleberbeschichtung (10) der Flachmaterialschicht (4) vollflächig aufgebracht ist.

9. Verband nach einem oder mehreren der vorstehenden Ansprüche, **dadurch gekennzeichnet, dass** die Kleberbeschichtung (10) der Flachmaterialschicht (4) auf Acrylatbasis ausgebildet ist.

10. Verband nach einem oder mehreren der vorstehenden Ansprüche, **dadurch gekennzeichnet, dass** die ablösbare Schutzschicht (14) auf Papier- oder Polymerfilmbasis, insbesondere auf Polyethylen-, Polypropylen- oder Polyesterbasis, ausgebildet ist.

11. Verband nach einem oder mehreren der vorstehenden Ansprüche, **dadurch gekennzeichnet, dass** die ablösbare Schutzschicht (14) eine Antihaftbeschichtung aufweist.

12. Verband nach einem oder mehreren der vorstehenden Ansprüche, **dadurch gekennzeichnet, dass** das Stützelement (8) auf Papier- oder Polymerfilmbasis ausgebildet ist, insbesondere auf Polyethylen-, Polypropylen-, Polyester- oder Polyamidbasis ausgebildet ist.

13. Verband nach einem oder mehreren der vorstehenden Ansprüche, **dadurch gekennzeichnet, dass** das Stützelement (8) eine Dicke von 10 - 500 µm, insbesondere 20 - 250 µm, insbesondere 25 - 100 µm aufweist.

14. Verband nach einem oder mehreren der vorstehenden Ansprüche, **dadurch gekennzeichnet, dass** das Stützelement (8) eine Antihaftbeschichtung auf seiner der Flachmaterialschicht zugewandten Seite aufweist.

## Claims

1. A trimmable wound bandage (2), namely film or foil bandage, for air- and negative pressure-tight closure of a wound space in negative pressure wound therapy, with a flat material layer (4), on film or foil basis, which effects the air- and negative pressure-tight closure with a frame forming support element (8) provided on the wound-averted side (6) of the flat material layer (4) which stabilizes the flat material (4) layer in areal extent during application of the wound bandage (2) and with a glue coating (10) provided on a wound-facing side (12) of the flat material layer (4)and with a detachable protective layer (4) covering the glue coating (10) until application of the bandage; and with multiple manually graspable, in particular tab-shaped or strip-shaped gripping regions (16) configured to not adhere to the glue coating (10), for facilitating detachment of the protective layer, wherein in circumferential direction (18) of the bandage and distributed over an outer circumference of the bandage, a multiplicity of gripping regions (16) is provided wherein the gripping regions are configured integral as one piece with the protective layer (14) and protrude transverse to the circumferential direction (18) of the bandage and the distance of the gripping regions (16) to each other in circumferential direction (18) of the bandage measured along a border edge of the bandage is at least 10 mm, and at most 80 mm, and wherein the frame forming support element (8) has sections (22) which extend strip- or web-shaped inwardly from an outer circumferential region (20) of the bandage and wherein the strip or web shaped extending sections meet each other.

2. The bandage of claim 1, **characterized in that** the distance of the gripping regions (16) to each other in circumferential direction of the bandage measured along a border edge of the bandage is at least 15 mm, in particular at least 20 mm, in particular at least 25 mm, and at most 60 mm.

3. The bandage of claim 1 or 2, **characterized in that** the strip or web shaped extending sections cross each other.

4. The bandage according to one or more of the preceding claims, **characterized in that** the support element (8) has preferably multiple weakening lines (26), in particular perforations which extend transverse to its extent along an outer circumference region(20) and/or transverse to the extent of its strip or web shaped extending sections (22) .

5. The bandage according to one or more of the preceding claims, the weakening lines (26) are spaced apart from each other by at least 30 mm, in particular at least 40 mm, in particular at least 50 mm, in particular at least 60 mm, and at most 150 mm, a particular at most 130 mm, in particular at most 100 mm.

6. The bandage according to one or more of the preceding claims, **characterized in that** the flat material layer (4) which effects the air- and negative pressure-tight closure, is configured transparent.

7. The bandage according to one or more of the preceding claims, the flat material layer (4) which effects the air- and negative pressure-tight closure, is formed on the basis of at least one member selected from the group of polyurethane, polyester, polypropylene, polyethylene, polyamide, polyvinyl chloride, and polyorganisiloxan or mixtures thereof.

8. The bandage according to one or more of the preceding claims, **characterized in that** the glue coating (10) of the flat material layer (4) is applied over its entire surface.

9. The bandage according to one or more of the preceding claims, **characterized in that** the glue coating (10) of the flat material layer (4) is formed on acrylate basis.

10. The bandage according to one or more of the preceding claims, **characterized in that** the detachable protective layer (14) is formed on paper-basis or polymer film-basis, in particular on polyethylene-, polypropylene- or polyester basis.

11. The bandage according to one or more of the preceding claims, **characterized in that** the detachable protective layer (14) has an antiadhesive coating.

12. The bandage according to one or more of the preceding claims, **characterized in that** the support element (8) is formed on paper basis or polymer film-basis, in particular on polyethylene-, polypropylene-, polyester- or polyamide-basis.

13. The bandage according to one or more of the preceding claims, **characterized in that** the support element (8) has a thickness of 10-500 µm in particular 10 - 250 µm, in particular 25 - 100 µm.

14. The bandage according to one or more of the preceding claims, **characterized in that** the support element (8) has an anti-adhesive coating on a side which faces the flat material layer.

## Revendications

1. Pansement découpable (2), à savoir pansement en film ou en feuille, destiné à être utilisé dans le traitement des plaies par pression négative pour fermer la zone d'une plaie de manière étanche à l'air et à la pression négative, comprenant une couche de matière plate (4) à base de film ou de feuille qui provoque la fermeture étanche à l'air et à la pression négative, un élément de soutien (8) formant cadre qui est prévu sur la face (6) de la couche de matière plate (4), laquelle est opposée à la plaie, et qui stabilise la couche de matière plate (4) lorsque le pansement (2) est appliqué en extension plate, ainsi qu'un revêtement adhésif (10) qui est prévu sur la face (12) de la couche de matière plate (4), laquelle est tournée vers la plaie, et une couche de protection (14) détachable qui couvre le revêtement adhésif (10) jusqu'à ce que le pansement soit appliqué, et des zones de préhension (16) saisissables à la main, en particulier en forme de languette ou de ruban qui n'adhèrent pas au revêtement adhésif (10) et au moyen desquelles un détachement de la couche de protection (14) du revêtement adhésif (10) est facilité, dans lequel une pluralité de zones de préhension (16) sont prévues qui sont réparties dans la direction circonférentielle (18) du pansement, sur la circonférence extérieure, et qui sont réalisées d'un seul tenant avec la couche de protection (14) et réalisées de manière à faire saillie vers l'extérieur transversalement à la direction circonférentielle (18) du pansement, et dans lequel la distance séparant les zones de préhension (16) les unes des autres dans la direction circonférentielle (18) du pansement, mesurée le long d'une arête marginale, est d'au moins 10 mm et de 80 mm tout au plus, et dans lequel ledit élément de soutien (8) formant cadre comprend des portions (22) étendues en forme de ruban ou d'entretoise qui s'étendent vers l'intérieur à partir d'une zone circonférentielle extérieure (20) du pansement, et ces portions (22) étendues en forme de ruban ou d'entretoise se rencontrent.

2. Pansement selon la revendication 1, **caractérisé par le fait que** la distance séparant les zones de préhension (16) les unes des autres dans la direction circonférentielle du pansement, mesurée le long d'une arête marginale, est de 15 mm au moins, en particulier de 20 mm au moins, en particulier de 25 mm au moins et de 60 mm tout au plus.

3. Pansement selon la revendication 1 ou 2, **caractérisé par le fait que** les portions (22) étendues en forme de ruban ou d'entretoise se croisent les unes les autres.

4. Pansement selon l'une ou plusieurs des revendications précédentes, **caractérisé par le fait que** ledit élément de support (8) présente de préférence une pluralité de lignes d'affaiblissement (26), en particulier de perforations, qui s'étendent transversalement à son extension le long d'une zone circonférentielle extérieure (20) et/ou transversalement à l'extension de ses portions (22) étendues en forme de ruban ou d'entretoise.

5. Pansement selon l'une ou plusieurs des revendications précédentes, **caractérisé par le fait que** les lignes d'affaiblissement (26) sont espacées les unes des autres de 30 mm au moins, en particulier de 40 mm au moins, en particulier de 50 mm au moins, en particulier de 60 mm au moins et de 150 mm tout au plus, en particulier de 130 mm tout au plus, en particulier de 100 mm tout au plus.

6. Pansement selon l'une ou plusieurs des revendications précédentes, **caractérisé par le fait que** ladite couche de matière plate (4) qui provoque la fermeture étanche à l'air et à la pression négative est réalisée de manière transparente.

7. Pansement selon l'une ou plusieurs des revendications précédentes, **caractérisé par le fait que** ladite couche de matière plate (4) qui provoque la fermeture étanche à l'air et à la pression négative est réalisée à base de polyuréthane, de polyester, de polypropylène, de polyéthylène, de polyamide, de polychlorure de vinyle ou de polyorganosiloxane ou de mélanges de ceux-ci.

8. Pansement selon l'une ou plusieurs des revendications précédentes, **caractérisé par le fait que** le revêtement adhésif (10) de la couche de matière plate (4) est appliqué sur toute la surface.

9. Pansement selon l'une ou plusieurs des revendications précédentes, **caractérisé par le fait que** le revêtement adhésif (10) de la couche de matière plate (4) est réalisé à base d'acrylate.

10. Pansement selon l'une ou plusieurs des revendications précédentes, **caractérisé par le fait que** la couche de protection (14) détachable est réalisée à base de papier ou de film de polymère, en particulier à base de polyéthylène, de polypropylène ou de polyester.

11. Pansement selon l'une ou plusieurs des revendications précédentes, **caractérisé par le fait que** la couche de protection (14) détachable est un revêtement anti-adhérent.

12. Pansement selon l'une ou plusieurs des revendications précédentes, **caractérisé par le fait que** ledit élément de support (8) est réalisé à base de papier ou de film de polymère, en particulier à base de polyéthylène, de polypropylène, de polyester ou de polyamide.

13. Pansement selon l'une ou plusieurs des revendications précédentes, **caractérisé par le fait que** ledit élément de support (8) présente une épaisseur comprise entre 10 et 500 µm, en particulier entre 20 et 250 µm, en particulier entre 25 et 100 µm.

14. Pansement selon l'une ou plusieurs des revendications précédentes, **caractérisé par le fait que** l'élément de support (8) présente un revêtement anti-adhérent sur sa face montrant vers la couche de matière plate.
